# EUROPEAN PATENT SPECIFICATION

(11) **EP 3 827 834 B1**
(45) Date of publication and mention of the grant of the patent: **11.03.2026**
(21) Application number: 20209244.1
(22) Date of filing: 23.11.2020
(51) Int. Cl.: A61K 36/185, A61K 36/484, A61K 36/63, A61K 36/73, A61K 36/76, A61K 36/15, A61K 36/41, A61K 36/46, A61K 36/53, A61K 36/539, A61K 36/66, A61K 36/704, A61K 36/734, A61K 36/79

(54) **EXTRACTION PROCESS OF COMPONENTS FROM A SUBSTRATE OF PLANT ORIGIN AND RESPECTIVE EXTRACTION PRODUCT**
EXTRAKTIONSVERFAHREN FÜR KOMPONENTEN PFLANZLICHER HERKUNFT UND DIE JEWEILIGEN EXTRAKTIONSPRODUKTE
PROCEDE D´EXTRACTION DES COMPOSES D´ORIGINE VEGETALE ET LES PRODUITS RESPECTIFS

(30) Priority: 25.11.2019 IT 201900022059
(43) Date of publication of application: 02.06.2021
(73) Proprietor: Naturalsalus Srl, 39100 Bolzano (BZ) (IT)
(72) Inventor: Morini, Angelo, 391 Bolzano (IT)
(74) Representative: Santoro, Sofia

(56) References cited:
- WO-A1-2018/004490
- CN-A- 101 199 595
- CN-A- 105 797 082

## Description

The present disclosure generally refers to a process for extracting components from a substrate of plant origin and to an extract thus obtained.

To date, the techniques for obtaining liquid extracts from substrates of plant origin, such as medicinal plants, herbs, roots and leaves, consist in the use of ethanol or hydroalcoholic mixtures, possibly added, in various ratios, with other substances (for example , glycerol or organic solvents). The extraction phase is usually preceded by drying and grinding of the substrate of plant origin, although, in some cases, it is also possible to use fresh substrates. Sometimes, the addition of excipients, such as antioxidants and antimicrobials, is also tolerated, with the aim of increasing the stability of the liquid extract over time.

The solid-liquid extraction techniques can be classified into:
1. simple maceration;
2. maceration under pressure;
3. maceration with ultrasounds;
4. percolation;

Although they are different methods, they have in common the characterization of the chemical profile of the plant and possible obtainment of a concentration of some specific fractions through the extraction, in a more or less intensive and selective way.

In the context of this disclosure, the term substrate of plant origin means a starting raw material of plant origin from which components with specific properties can be extracted. For example, it can be a vegetable raw material, in herbaceous form, or shrub, or a fruit or a flower, a root, an inflorescence, a bud, and the like, i.e. any component of plant origin or a derivative thereof. In the following these components may also be referred to as active components. A so-called extract obtained after extraction of the substrate is hereinafter referred to as an extraction product, or extract.

Preferably these are components having antioxidant capacity or surfactant or solvation action.

An extraction phase is to be understood as a phase wherein the solid substrate is placed in contact with an extraction solvent, i.e. a substance, preferably a substance in a fluid form, which in contact with the substrate is able to extract, i.e. that is, take out one or more components and transfer them to the hydroalcoholic extraction solvent. The extraction solvent is sometimes referred to in this application as an extraction vehicle.

To date, the extraction normally takes place on a substrate of fresh plant origin or more frequently on a dried substrate, as it is available throughout the year without being bound only to the seasonality of the harvest.

With a substrate consisting of plants / herbs, the active components derived from the plants / herbs are extracted via the solvent.

For example, extraction processes from plant substrates are known from WO 2018/004490 A1, CN 105 797 082 A, and CN 101 199 595 A.

It is also known to prepare extraction products of multiple plant substrates in order to have a combination of active components available for the preparation of a certain final composition. The main active components of the plants that are extracted are antioxidant components.

A technical problem of the extraction of many components from a plurality of plant substrates is mainly linked to the fact that at the end of all the single extractions of the single substrates there are a plurality of hydroalcoholic solvent extracts which are joined. These extracts, once combined, lead to sometimes excessive dilution of the components.

There is therefore a need to concentrate the extraction products in order to have extracts with a sufficiently high amount of active component in the extraction product, so that the active component is effective even with small doses of the final composition.

To this end, at the end of each extraction of a respective plant substrate, the extraction products are concentrated so as to eliminate solvent and so that the final product is not excessively diluted. At this point, the single concentrated extraction products are combined into a single final composition.

Although the known process is advantageous from many points of view, it requires concentration steps for eliminating excess solvent. These concentration phases, in addition to requiring additional processing phases and lengthening of production times, can sometimes be carried out in extreme and forced conditions, for example evaporation, with the risk of damaging the active components and reducing the range of active components, to reduce to only a part of them.

A technical problem underlying this disclosure resides in providing an extraction process that can make it possible to obtain a concentrated extract of active components, without or with minimal need to concentrate the extraction product after extraction.

This problem is solved by a process, a use, an extract and a preparation according to the respective independent claims.

Secondary characteristics of the subject-matter of this disclosure are defined in the respective dependent claims.

In particular, according to an aspect of the present disclosure, a process is made available which provides for carrying out at least a first extraction phase, a second extraction phase. From the first extraction phase a first extraction product is obtained and said first extraction product is all used as an extraction vehicle or solvent to carry out the second extraction phase, on a second substrate.

In practice, the extraction product is used as a whole as an extraction vehicle for a second substrate. Therefore, the vehicle or extraction solvent of the second extraction phase includes extraction components that can interact with the second substrate and improve extraction or make extraction more effective.

Still more particularly in accordance with the present disclosure, an extraction process is made available for extracting with hydroalcoholic solvent one or more components from at least two substrates of plant origin, wherein the process comprises
at least a first extraction phase and a second extraction phase, wherein the first extraction phase involves preparing a hydroalcoholic solvent and treating a first plant substrate with said hydroalcoholic solvent to transfer one or more first components of the first plant substrate into said hydroalcoholic solvent obtaining a first extraction product including said hydroalcoholic solvent and said one or more first components,
and collecting the first extraction product and using the first extraction product as primary extraction solvent,
the second extraction phase involves preparing the primary extraction solvent and treating a second plant substrate with said primary extraction solvent in order to transfer one or more second components of the second plant substrate into said primary extraction solvent, obtaining a second product of extraction including said hydroalcoholic solvent and said one or more first components, and said one or more second components;
and collecting the second extraction product or second extract,

The process includes a third extraction phase following the second extraction phase. The third extraction phase involves preparing the secondary extraction solvent and treating a third plant substrate with said secondary extraction solvent in order to transfer one or more third components of the third plant substrate into said secondary extraction solvent obtaining a third extraction product or third extract including said one or more first components, said one or more second components and said one or more third components
and collecting said third extraction product.

Even more preferably a subsequent extraction phase is provided, carried out on a subsequent plant substrate using the third extraction product as a tertiary extraction solvent, and wherein a further subsequent extraction phase is carried out in cascade on a subsequent plant substrate using a product of extraction of the previous extraction phase as extraction solvent of said subsequent extraction phase.

Basically, in a cascade mode, an extraction product can be used several times on many other substrates to carry out a so-called cascade extraction or iterative extraction wherein the same extraction product is used as an extraction vehicle for a subsequent extraction, on a different substrate not yet extracted.

In other words, the present disclosure refers, in general, to a solid-liquid extraction process called "cascade", which allows the components to be concentrated by successive cascade extractions in a starting solvent (possibly only by adding few refills), and therefore also reduce the use of solvents. It follows that in addition to reducing the need to concentrate a final product, the process therefore allows a substantial reduction in the use of solvents compared to similar procedures, performed with one of the traditional procedures described above, and therefore, not in "cascade" mode.

The aforementioned first substrate of plant origin from which the primary extraction solvent is obtained is selected from the group including Brassica Oleracea Var Italica, Crataegus Oxyacantha auct., Glycyrrhiza Glabra L. e Ptychopetalum Olacoides Benth, or a combination of some components.

This group is included in Table 1 below.

The second or subsequent substrate, such as third substrate, fourth substrate or other subsequent substrate, is preferably selected from the group including Birch pubescens, Brassica Oleracea Var Italica, Crataegus Oxyacantha auct., Eucommia Ulmoides Oliv., Filipendula Ulmaria Max., Fraxinus Excelsior, Glycyrrhiza Glabra_L., Harpagophytum Procubens D.C., Hippophae Rhamnoides L., Melissa Officinalis L., Moringa Oleifera Lamk, Olea Europaea L., Papaver Rhoeas L., Passiflora Incarnata L., Poligonum Multiflorum Thunb., Ptychopetalum_Olacoides_LBenthali_L., Rhodosola_Benthali_L Schisandra_Chinensis_baill., Turnera_Aphrodisiaca_Willd., Urtica_Dioica L., or a combination of some components.

This group is included in Table 2 below.

Therefore, the present disclosure consists in the use of a "primary extraction solvent", obtained for example by the extraction of components with hydroalcoholic solvent from a first substrate of plant origin among those chosen among the "substrates of plant origin" listed in Table 1, to extract one or more components from at least a second "substrate of plant origin", wherein said second substrate is chosen from those listed in Table 2. This extraction procedure can be expanded in "cascade" mode, that is to say the liquid extract obtained from an extraction procedure, becomes the solvent for a subsequent extraction phase on the same or other substrates of plant origin.

**Table 1. List of substrates of plant origin to be used as primary extraction solvent Legend: supporting technologies for extraction we mean the use of: M: maceration; US: ultrasound (Frequency 24 -45kHz, Power: 200 - 1500W).**

| Vegetable matrix | Relative humidity (UR%) | Grain size (mm) | Solvent (100%) | | Extraction temperature (°C) | Extraction time (hours) | Supporting technologies |
|---|---|---|---|---|---|---|---|
| | | | Water (%) | Ethanol (%) | | | |
| | | | | | | | |
| *Brassica Oleracea Var Italica* | 0-45 | 0.1 - 20 | 50 - 100 | 0-50 | 20 - 60 | 0.25 - 48 | M, US |
| *Crataegus Oxyacantha auct.* | 0-45 | 0.1 - 20 | 50 - 100 | 0-50 | 20 - 60 | 0.25 - 48 | M, US |
| *Glycyrrhiza Glabra L.* | 0-45 | 0.1 - 20 | 50 - 100 | 0-50 | 20 - 60 | 0.25 - 48 | M, US |
| *Ptychopetalum Olacoides Benth.* | 0-45 | 0.1 - 20 | 50 - 100 | 0-50 | 20 - 60 | 0.25 - 48 | M, US |

**Table 2. List of substrates of plant origin to be used as secondary substrates for obtaining plant extracts with surprising and advantageous extraction yields in phytocompounds with antioxidant action.**

| **Substrate of plant origin** | **Anatomical part** | **Physical state** |
|---|---|---|
| *Birch pubescens* | Limpha | Liquid |
| *Brassica Oleracea Var Italica* | Sprouts | Solid |
| *Crataegus Oxyacantha auct.* | Folium | Solid |
| *Eucommia Ulmoides Oliv.* | Cortex | Solid |
| *Filipendula Ulmaria Max.* | Flos | Solid |
| *Fraxinus Excelsior* | Folium | Solid |
| *Glycyrrhiza Glabra_L.* | Radix | Solid |
| *Harpagophytum Procubens DC* | Radix | Solid |
| *Hippophae Rhamnoides L.* | Fructus | Solid |
| *Melissa Officinalis L.* | Flos et Folium | Solid |
| *Moringa Oleifera Lamk* | Folium | Solid |
| *Olea Europaea L.* | Folium | Solid |
| *Papaver Rhoeas L.* | Flos | Solid |
| *Passiflora Incarnata L.* | Herba cum floribus | Solido |
| *Poligonum Multiflorum Thunb.* | Radix | Solid |
| *Ptychopetalum_Olacoides_Benth.* | Lignum | Solid |
| *Rhodiola_Rosea_L.* | Radix | Solid |
| *Salix_Alba_L.* | Cortex | Solid |
| *Schisandra_Chinensis_baill.* | Fructus | Solid |
| *Turnera_Aphrodisiaca_Willd.* | Folium | Solid |
| *Urtica_Dioica L.* | Folium | Solid |

In other words, the process object of the present disclosure includes, at a minimum, two extraction phases, where the first phase consists in the hydroalcoholic extraction of one of the substrates of plant origin, such as for example those listed in Table 1, while the second, consists in using this liquid extract as a solvent for the extraction of one of the substrates of plant origin, such as those listed in Table 2. In practice, from the first extraction phase, a first hydroalcoholic liquid extract is obtained. This is then used, in whole or in part, as a solvent for the second extraction phase.

The product of each extraction, performed with the technique described in this claim, can be used several times on many other substrates of plant origin, to carry out the so-called "cascade" extraction. In this type of extraction, the same extraction product is used as a solvent for extraction in a subsequent extraction phase on a different substrate than the first, for example among those present in Table 2.

Preferably the substrates of plant origin such as those of table 1 or of table 2, are chosen so as to contain compounds with antioxidant action and/or compounds with surfactant or solvation action. It follows that after the first extraction, the primary extraction agent and/or the secondary extraction solvent are enriched in compounds with antioxidant action and/or compounds with surfactant or solvation action. It follows that the primary extraction solvent, obtained from the substrates and operating conditions indicated in Table 1, may include substances that can improve or make the extraction of the components of the second substrate more effective.

In other words, the first plant substrate preferably includes at least one component having antioxidant capacity and/or one component having solvation capacity or surfactant action, and in said first extraction phase said at least one component with antioxidant capacity and/or said component with solvation capacity or surfactant action are at least partially transferred into said hydroalcoholic solvent.

The second plant substrate also preferably includes at least one component having antioxidant capacity and/or one component having solvation capacity or surfactant action, and in said second extraction phase said at least component with antioxidant capacity and/or component having solvation capacity or surfactant action are at least partially transferred into said primary extraction solvent.

In this way the antioxidant component of a primary extraction solvent is able to counteract the oxidative tendency of other components present in the second substrate or in other substrates, and therefore to obtain a final extract, not only concentrated, but also with a greater quantity. of non-oxidized compounds, and the same advantage can be obtained for subsequent extractions. Similarly, if a substrate includes a compound having solvation capabilities, such compound can facilitate the solubility and transfer to the extraction liquid of compounds contained in substrates used in subsequent extraction phases.

Preferably, the extraction can take place by maceration, infusion or decoction, for example within the operating conditions indicated in Table 1. Preferably, these techniques can be supported by the use of ultrasound, in order to reduce the extraction times. In particular, as regards the first extraction phase, mainly aqueous solvents or hydroalcoholic mixtures with a alcohol content between 20% v/v and 80% v/v are preferably used, preferably between 30% v/v and 70% v/v more preferably still between 40% and 60%, or 50% v/v.

Extraction times preferably take place within 48 hours for substrate of plant origin. That is, the first substrate of plant origin is preferably immersed in the hydroalcoholic solvent or liquid extract obtained from the previous extraction, at a temperature preferably between 20 °C and 60 °C, and preferably at 40 °C. At the end of the procedure, after waiting for at least 0.25-48 hours, preferably within 24 hours, and, in the case of extraction techniques with ultrasound support, preferably within 6 hours, the extract is filtered on a suitable sieve or sieve covered by filter paper, by percolation or by vacuum filtration. Subsequently, the solid substrate of plant origin still soaked in solvent can be squeezed by hand or mechanically. The liquid obtained from this squeezing operation is then added to the liquid extract.

The extract is then ready to be used as a solvent for a second substrate of plant origin. The operation can be repeated in "cascade" mode, on a third substrate of plant origin, and preferably so on. It should be understood that a person skilled in the art can apply other known extraction techniques, applying the cascade sequence described here.

The "cascade" extraction process, according to this disclosure, has the obvious advantage that only the same primary extraction solvent is always used. In particular, the "cascade" extraction process, reusing the same primary hydroalcoholic solvent, allows, in each cascade extraction phase, to enrich the content of phytocompounds in the primary extract, on the other hand, not requiring the use of further hydroalcoholic solvents with the exception of the first extraction. Overall, this results in the possibility of avoiding a concentration step of the extraction product as well as an evident saving of organic solvents.

Furthermore, if the substrate of plant origin contains antioxidant compounds, the extraction yield in phytocompounds of the second substrate of plant origin, or of the following ones, can be improved with respect to that obtainable by extraction with the same hydroalcoholic solvent. This improvement is obtainable thanks to the presence of phytocompounds with antioxidant action already present in the primary extraction solvent, which allow to increase the stability of the phytocompounds extracted during the second or subsequent extraction phase.

It is possible to improve the extraction yield in phytocompounds of the second substrate of plant origin, or of the following ones, also thanks to the presence of phytocompounds with surfactant action, i.e. able to lower the surface tension of the aqueous solvent, at the solid-liquid interface, increasing the extraction capacity of the solvent itself.

It can be understood that the process according to the present disclosure can be designed or developed according to the finished product to be obtained, so as to optimize the choice of substrates and components that can be extracted from such substrates, and of the possible interaction that an extraction product may have with a substrate to optimize subsequent extraction.

For example, one can start from an extraction, for example maceration / percolation on a plant, or other substrate. Then this first extraction product is used to extract the next plant, or other substrate, and so on until the finished product is composed.

Preferably, the extraction occurs mainly through maceration, and even more preferably an ultrasonic maceration which, in addition to reducing the maceration times, has an inductive property on the enzymatic activity of the plants and therefore promotes a certain reactivity for which it activates the extracted making it more suitable for subsequent extraction

Preferably the plants used as substrate are chosen from one or more of the following families: Asteraceae; Betulaceae; Brassicacee; Crassulaceae; Elaeagnaceae; Eucommiaceae; Fabaceae; Lamiaceae; Lauraceae; Moringaceae; Olacaceae; Papaveraceae; Passifloraceae; Pedaliaceae; Polygonaceae; Rosaceae; Salicaceae; Sapindaceae; Schisandraceae; Schisandraceae; Turneraceae; Urticaceae.

In one embodiment the plants used as substrate are selected from one or more of the following plants: Acorus calamus L.; Angelica archangelica L.; Arctostaphylos uva-ursi (L.) Sprengel; Birch pubescens limpha; Brassica oleracea var. Italica folia (Sulforaphane); Carica papaya L.; Carum carvi L.; Crataegus Oxyacantha auct. Folium; Equisetum arvense L.; Eucommia Ulmoides Oliv. Cortex; Ficus carica L.; Filipendula ulmaria Max. flos; Foeniculum vulgare Miller; Fraxinus excelsior folium; Glycyrrhiza glabra L.; Glycyrrhiza glabra L. radix; Harpagophytum procubens D.C. radix; Hieracium pilosella L.; Hippophae rhamnoides L. Fructus; Litsea cubeba; Malva sylvestris L.; Melissa officinalis L. flos et folium 10%; Moringa oleifera lamk. Folium; Nasturtium officinale R. Brown; Olea Europaea L. Folium; Papaver Rhoeas L. Flos; Passiflora incarnata L. herba cum floribus 50%; Paullinia Cupana Kunt semen; Poligonum multiflorum thunb. Radix; Ptychopetalum Olacoides Benth. Lignum; Rhodiola rosea L. radix 10%; Salix alba L. cortex; Schisandra chinensis baill. Fructus; Sempervivum tectorum L.; Solidago virgaurea L.; Stevia rebaudiana; Tabebuia avellanedae Lorenz; Turnera Aphrodisiaca Willd. Folia; Urtica Dioica L. Folium

In one embodiment, plants of the following families are extracted sequentially, i.e. in cascade mode: Brassicacee; Schisandraceae; Elaeagnaceae; Moringaceae; Polygonaceae. Even more preferably, the following plants are extracted in cascade mode or sequentially: Brassica oleracea var. Italica folia (Sulforaphane); Schisandra chinensis baill. Fructus; Hippophae rhamnoides L. Fructus; Moringa oleifera lamk. Folium; Poligonum multiflorum thunb. Radix. The final extraction product can have an antioxidant function and purifying functions for the organism.

In one embodiment, plants of the following families are extracted sequentially, i.e. in cascade mode: Betulaceae; Fabaceae; Salicaceae; Rosaceae; Oleaceae; Pedaliaceae; Asteraceae. Even more preferably, the following plants are extracted in cascade mode or sequentially: Birch pubescens limpha; Glycyrrhiza glabra L. radix; Salix alba L. cortex; Filipendula ulmaria Max. flos; Fraxinus excelsior folium; Harpagophytum procubens D.C. radix; Stevia rebaudiana. The final extraction product may have properties against rheumatic and muscle pain.

In one embodiment, plants of the following families are extracted sequentially, i.e. in cascade mode: Rosaceae; Passifloraceae; Lamiaceae; Crassulaceae; Lauraceae; Asteraceae. Even more preferably, the following plants are extracted in cascade mode or sequentially: Crataegus Oxyacantha auct. Folium; Passiflora incarnata L. herba cum floribus; Melissa officinalis L. flos et folium; Rhodiola rosea L. radix; Litsea cubeba; Stevia rebaudiana. The final extraction product may have properties against anxiety and palpitations, sleeplessness at night.

In one embodiment, plants of the following families are extracted sequentially, i.e. in cascade mode: Olacaceae; Turneraceae; Schisandraceae; Polygonaceae; Sapindaceae. Even more preferably, the following plants are extracted in cascade mode or sequentially: Ptychopetalum Olacoides Benth. Lignum; Turnera Aphrodisiaca Willd. Folia; Schisandra chinensis baill. Fructus; Poligonum multiflorum thunb. Radix; Paullinia Cupana Kunt semen. The final product of extraction can be useful as a tonic for the body.

In one embodiment, plants of the following families are extracted sequentially, i.e. in cascade mode: Eucommiaceae; Urticaceae; Oleaceae; Papaveraceae. Even more preferably, the following plants are extracted in cascade mode or sequentially: Eucommia Ulmoides Oliv. Cortex; Urtica Dioica L. Folium; Olea Europaea L. Folium; Papaver Rhoeas L. Flos. The final extraction product can be useful for controlling hypertension.

In one embodiment, plants of the following families are extracted sequentially, i.e. in cascade mode: Ericacee; Asteracee; Malvaceae; Asteraceae.

Even more preferably, the following plants are extracted in cascade mode or sequentially: Arctostaphylos uva-ursi (L.) Sprengel; Solidago virgaurea L.; Malva sylvestris L.; Hieracium pilosella L.

In one embodiment, plants of the following families are extracted sequentially, i.e. in cascade mode: Crassulaceae; Equisetaceae; Brassicaceae. Even more preferably, the following plants are extracted in cascade mode or sequentially: Sempervivum tectorum L.; Equisetum arvense L.; Nasturtium officinale R. Brown.

In one embodiment, plants of the following families are extracted sequentially, i.e. in cascade mode: Apiaceae; Apiaceae; Apiaceae; Acoraceae; Moraceae; Leguminosae. Even more preferably, the following plants are extracted in cascade mode or sequentially: Foeniculum vulgare Miller; Carum carvi L.; Angelica archangelica L.; Acorus calamus L.; Ficus carica L.; Glycyrrhiza glabra L.

In one embodiment, plants of the following families are extracted sequentially, i.e. in cascade mode: Apiaceae; Bignoniaceae; Caricaceae. More preferably, the following plants are extracted in cascade, i.e. sequentially: Angelica archangelica L.; Tabebuia avellanedae Lorenz; Carica papaya L.

Other advantages, characteristics and methods of use of the object of the present disclosure will become evident from the following detailed description of some of its preferred embodiments, given by way of non-limiting example. However, it is evident that each embodiment can have one or more of the advantages listed above; in any case, it is not however required that each embodiment have all the listed advantages simultaneously.
Reference will also be made to the enclosed
- Figure 1, which shows the heat measured with a calorimetric method of the samples of example 11 below.
- Figure 2 which shows a portion of the HPLC chromatogram in relation to example 2 below.
- Figure 3 which shows a portion of the HPLC chromatogram in relation to example 6 below.
- Figure 4 in which the result obtained for example 6 is illustrated with another graph.

### EXAMPLE 1

It was planned to carry out a "cascade" extraction of five substrates of plant origin, schematically indicated as "A", "B", "C", "D", "E". The resulting liquid extract at the end of the procedure covered by this claim is referred to as "ABCDE" or final extraction product. The final extraction product can be obtained as follows:
1) The substrate of plant origin "A" is collected and used fresh, or dried until it reaches a relative humidity level between 0 and 45%. Subsequently, the substrate "A" is used as it is, or ground with mechanical mills, operating by crushing or cutting or in any case with other shredding technology, until grains of a few millimeters or fine dust are obtained;
2) The substrate "A", suitably processed as described in 1), is then extracted with hydroalcoholic solvent. The extraction can take place by infusion, decoction, maceration, with or without ultrasound support, according to the conditions described in Table 1 above, or at a temperature of 20-60 °C, for 0,25 - 48 h.
3) the liquid obtained from plant "A", also called extraction product A, will be used as the first extraction vehicle for the extraction of the substrate of plant origin B (substrate B, or second substrate), obtaining the extract AB ( AB extraction product) which will therefore have a complex composition capable of interacting in a specific way on the subsequent extraction phases; The substrate of plant origin "B" is used fresh, or dried until it reaches a relative humidity level between 0 and 45%. Subsequently, the substrate "B" is used as it is, or ground with mechanical mills, operating by crushing or cutting or in any case with other shredding technology, until grains of a few millimeters or fine dust are obtained;
3) the extracted product "AB" will therefore be used to extract the plant "C" and "D" which have the same balsamic time and are therefore collected and macerated together, to form the "ABCD" extraction product. Similarly to "A" and "B", the substrates of plant origin "C" and "D" can be used fresh, or dried until they reach a relative humidity level between 0 and 45%. Subsequently, the substrates are used as they are, or ground with mechanical mills, operating by crushing or cutting or in any case with other shredding technology, until grains of a few millimeters or fine dust are obtained;
4) the "ABCD" extraction product or ABCD solution, which will therefore have the characteristics of each of the components, but also those synergies (or chemical complexes) that are formed thanks to the sequential interaction of the various plants.
5) the "ABCD" extraction product or ABCD solution is used for the extraction process of the last plant "E", to form the final extraction product. With this last phase of the procedure the procedure is concluded. The resulting liquid extract will consist of a content of phytocompounds with a higher antioxidant action than the liquid extract obtained by weighted mixing of the single extracts "A", "B", "C", "D", "E", obtained individually by the use of a hydroalcoholic solution.

With a "cascade" process according to the present disclosure, a liquid extract is produced with a much lower volume of hydroalcoholic solvent than the same extract obtained by mixing five single extractions. With the "cascade" process, assuming a ratio between solute and solvent, i.e. between substrate of plant origin and hydroalcoholic solvent, equal to 1:10, 10 kg of hydroalcoholic solvent are required for the processing of 1 kg of substrate "A" . To proceed with the extraction of the second substrate "B", the extraction product A will be reused as a solvent. Therefore, it will not be necessary to consume any additional solvent, except for the top-up required to compensate for the losses due to the partial evaporation of the solvent or the loss of solvent soaked on the solid residue. The same will be said for the extraction of the substrate "CD" and "E". In total, the extraction process object of this claim requires 10 kg of hydroalcoholic solvent for the extraction of 5 kg of substrates of plant origin. Instead, the same extraction procedure carried out in the traditional way would require the use, overall, of 50 kg of hydroalcoholic solvent for the similar extraction of 5 kg of substrates of plant origin, keeping the ratio of 1:10 between solvent and solute constant.
In fact, with the "cascade" process according to the present disclosure, the reuse of the hydroalcoholic solvent during the extraction of each substrate of plant origin allows to progressively enrich the solvent, avoiding the use of fresh hydroalcoholic solvent, unless any top-ups that may be required to compensate for the losses due to the partial evaporation of the solvent during the extraction process or to the loss of solvent soaked in the solid residue.

In other words, with the process according to the present disclosure, it is possible to have a much lower use of solvent to obtain a concentrated extract and it does not require evaporation to concentrate it.

In practice, if an ABCDE mixture of plants in equal parts is made, in the traditional way we will have to extract each plant individually and then mix a portion of the extract. With the process according to the present disclosure, it is extracted progressively enriching the same solvent with each plant and we already obtain the final mixture. For example, in a traditional extraction process, one proceeds as follows. For example, A is extracted in a liter, for example with a 20% extraction. 1: 5, and extract B in another liter, for example with a 20% extraction. 1: 5. To obtain 1 liter of finished product ABCDE I mix 20% of A + B + C + D + E, wherein each plant enters with 4% (each extraction product at 20% of the extract 1: 5).

Thanks to the process according to the present disclosure, if a liter of A is extracted with an extraction product at 20%. 1: 5); the resulting extraction product is used to extract B (always 1: 5); and again the resulting extraction product AB is used to extract C; and the resulting extraction product ABC is used to extract D; and finally the resulting extraction product ABCD is used to extract E. In the end, 1 liter of ABCDE is obtained (compared to 5 liters traditional extraction) wherein each plant enters at 20% (5 times more concentrated than the traditional).

### EXAMPLE 2

A liquid extract of Crataegus oxyacantha auct., Commonly called hawthorn, is obtained starting from the dry plant substrate, preliminarily lyophilized and reduced to powder with a hammer mill, and finally standardized for the size of the powders by air sieving with lower meshes at 800 µm. A mass of about 100 g of dry plant substrate is then mixed with 1 L of hydroalcoholic solution at 60% v/v in alcohol (solute: solvent ratio equal to 1:10) and extracted at 40 °C in an assisted glass reactor with an ultrasonic sonotrode for a period of 40 min. The liquid extract obtained is then used as a second solvent for the extraction of 100 g of Passiflora incarnata L., using a procedure identical to the previous one. The liquid extract obtained is then used as a third solvent for the extraction of 100 g of *Melissa officinalis L.,* using a procedure identical to the previous one. Finally, the liquid extract obtained is then used as a fourth solvent for the extraction of 100 g of *Rhodiola* rosea *L.,* using a procedure identical to the previous one.

The single extracts of each plant substrate obtained using the hydroalcoholic solvent, the final extract obtained with the *"cascade"* procedure and the extract obtained by mixing the single extracts in equal proportions to those obtained with the "cascade" procedure, were analyzed with DPPH assay for the measurement of antioxidant activity. The results are reported in the following Table 3.

**Table 3**

| **Label** | **Name** | **% in the formulation** | **mM/g** |
|---|---|---|---|
| A | *Crataegus oxyacantha auct.* | 30% | 3.4 ± 0.3 |
| B | *Passiflora incarnata L.* | 50% | 0.1 ± 0.1 |
| C | *Melissa Officinalis L.* | 10% | 5.8 ± 0.5 |
| D | *Rhodiola rosea L.* | 10% | 7.6 ± 0.3 |
| **Seq.** | **"Cascade" sequence** | | **2.2 ± 0.3** |
| Mix | Weighted average of single extracts | | 1.2 ± 0.2 |

As can be seen from the antioxidant activity data, the extract obtained with the cascade sequence (Seq.) presents an antioxidant power almost double compared to that obtained by carefully mixing the individual single extracts (Mix).

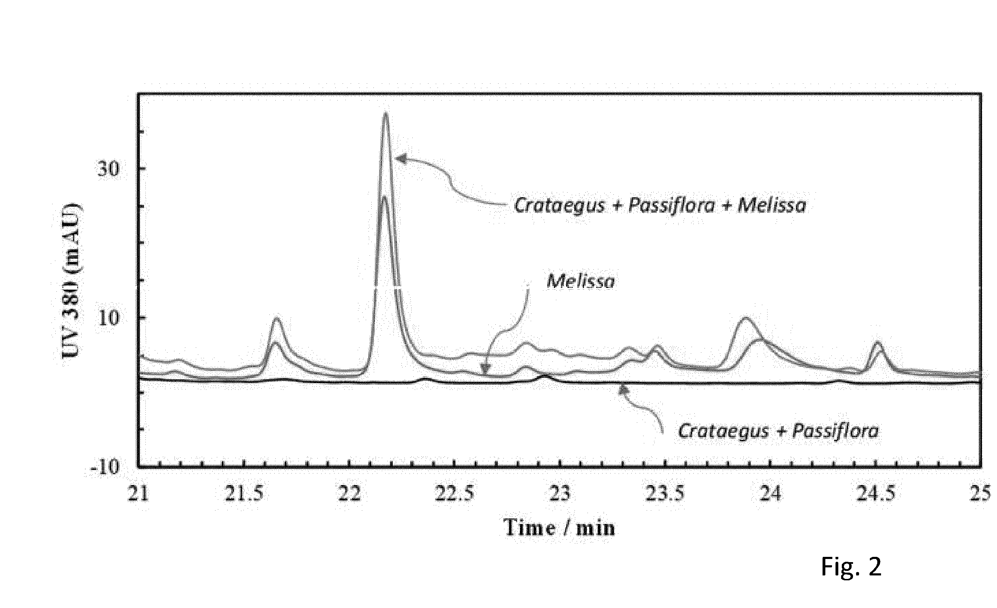

Figure 2 also shows the profile of compounds extracted by HPLC with a diode matrix detector. The absorbance signal recorded at the wavelength of 380 nm. In particular, Figure 2 shows an HPLC obtained with a diode array detector at a wavelength of 360 nm. The three traces refer respectively to the liquid extract of Crataegus oxyacantha auct. and Passiflora incarnata_L. and, subsequently, to the extract obtained using the previous liquid extract to extract Melissa officinalis L., and then Rhodiola rosea L.

In particular, HPLC liquid chromatography with UV detector was used to compare the chemical profile of the liquid extract of Melissa officinalis obtained using the "cascade" extract of Crataegus oxyacantha and Passiflora incarnata as a solvent, with the hydroalcoholic extract of Lemon balm. officinalis. The comparison shows that the Melissa officinalis extract obtained with the cascade process has a much higher signal than that obtained from the hydroalcoholic extract.

### EXAMPLE 3

A liquid extract of Crataegus oxyacantha auct., Commonly called hawthorn, is obtained starting from the dry plant substrate, preliminarily lyophilized and reduced to powder with a hammer mill, and finally standardized for the size of the powders by air sieving with lower meshes at 800 µm. A mass of about 100 g of dry plant substrate is then mixed with 1 L of hydroalcoholic solution at 60% v/v in alcohol (solute: solvent ratio equal to 1:10) and extracted at 40 °C in an assisted glass reactor with an ultrasonic sonotrode for a period of 40 min. The liquid extract obtained is then used as a second solvent for the extraction of 100 g of Eucommia ulmoides oliv, using a procedure identical to the previous one. The liquid extract obtained is then used as a third solvent for the extraction of 100 g of Urtica Dioica L., using a procedure identical to the previous one. The liquid extract obtained is then used as a fourth solvent for the extraction of 100 g of Olea Europaea L., using a procedure identical to the previous one. Finally, the liquid extract obtained is then used as the fifth solvent for the extraction of 100 g of Rhodiola rosea L., using a procedure identical to the previous one.

The single extracts of each plant substrate obtained using the hydroalcoholic solvent, the final extract obtained with the "cascade" procedure and the extract obtained by mixing the single extracts in equal proportions to those obtained with the "cascade" procedure, were analyzed with DPPH assay for the measurement of antioxidant activity. The results are reported in the following Table 4.

**Table 4**

| **Label** | **Name** | **% in the formulation** | **mM / g** |
|---|---|---|---|
| A | *Crataegus Oxyacantha auct.* | 41% | 4.3 ± 0.5 |
| B | *Eucommia Ulmoides Oliv.* | 20% | 0.1 ± 0.1 |
| C | *Urtica Dioica L.* | 24% | 1.7 ± 0.3 |
| D | *Olea Europaea L.* | 9.8% | 1.7 ± 0.2 |
| | *Papaver Rhoeas L.* | 5.1% | 2.8 ± 0.4 |
| **Seq.** | **"Cascade" sequence** | | **1.7 ± 0.2** |
| Mix | Weighted average of single extracts | | 1.0 ± 0.1 |

As can be seen from the antioxidant activity data, the extract obtained with the cascade sequence (Seq.) presents an antioxidant power almost double compared to that obtained by carefully mixing the individual single extracts (Mix).

### EXAMPLE 4

A liquid extract of Glycyrrhiza glabra L. is obtained starting from the dry plant substrate, previously freeze-dried and powdered by a hammer mill, and finally standardized for the size of the powders by air sieving with meshes of less than 800 µm. A mass of about 100 g of dry plant substrate is then mixed with 1 L of hydroalcoholic solution at 60% v/v in alcohol (solute: solvent ratio equal to 1:10) and extracted at 40 °C in an assisted glass reactor with an ultrasonic sonotrode for a period of 40 min. The liquid extract obtained is then used as a second solvent for the extraction of 100 g of Salix alba L., using a procedure identical to the previous one. The liquid extract obtained is then used as a third solvent for the extraction of 100 g of Filipendula ulmaria max., using a procedure identical to the previous one. The liquid extract obtained is then used as a fourth solvent for the extraction of 100 g of Fraxinus Excelsior, using a procedure identical to the previous one. Finally, the liquid extract obtained is then used as the fifth solvent for the extraction of 100 g of Harpagophytum procubens, using a procedure identical to the previous one.

The single extracts of each plant substrate obtained using the hydroalcoholic solvent, the final extract obtained with the "cascade" procedure and the extract obtained by mixing the single extracts in equal proportions to those obtained with the "cascade" procedure, were analyzed with DPPH assay for the measurement of antioxidant activity. The results are reported in the following Table 5.

**Table 5**

| **Label** | **Name** | **% in the formulation** | **mM / g** |
|---|---|---|---|
| A | *Glycyrrhiza Glabra L.* | 11% | 0.9 ± 0.1 |
| B | *Salix Alba L.* | 38% | 3.6 ± 0.3 |
| C | *Filipendula Ulmaria Max.* | 30% | 7.1 ± 1.1 |
| D | *Fraxinus Excelsior* | 11% | 3.5 ± 0.4 |
| E | *Harpagophytum Procubens D.C.* | 11% | 1.4 ± 0.2 |
| **Seq.** | **"Cascade" sequence** | | **2.6 ± 0.2** |
| Mix | Weighted average of single extracts | | 1.6 ± 0.2 |

As can be seen from the antioxidant activity data, the extract obtained with the cascade sequence (Seq.) presents an antioxidant power almost double compared to that obtained by carefully mixing the single extracts (Mix). EXAMPLE 5 A method based on the substrates according to table 6 was prepared. As substrates were used in the order: Broccoli Sprouts; Schisandra chinensis whole fruit; Sea Buckthorn fruits; Moringa tea leaves. A hydroalcoholic solution is used as a solvent consisting of:
- Water 600 gr
- Alcohol 96% 400 gr

**TABLE 6**

| ***Raw material*** | ***Botanical name*** | ***Family*** | ***Amount gr x 1 kg*** |
|---|---|---|---|
| Broccolo Sprouts | Brassica oleracea var. Italica folia (Sulforaphane) | Brassicacee | 250 |
| Schisandra chinensis whole fruits | Schisandra chinensis baill. Fructus | Schisandraceae | 250 |
| Olivello Spinoso fruits | Hippophae rhamnoides L. Fructus | Elaeagnaceae | 250 |
| Moringa tea leaves | Moringa oleifera lamk. Folium | Moringaceae | 250 |

It then operates making a maceration of the moringa leaves in ultrasound for 48 hours 54 degrees. The residue is filtered, squeezed and then the solvent is used to extract schisandra fruit, 24 hours 57 degrees. The residue is filtered and squeezed and then the solvent is used to extract sea-buckthorn fruit, 24 hours 57 degrees. The residue is filtered and squeezed and used by maceration on broccoli sprouts under turboemulsifier in cycles of 15 minutes every hour for 72 hours (which in this way release the greatest amount of sulforaphane). This extract will then be finished with filtration and squeezing of the residue and can be used as an ingredient to compose the final product by mixing it with honey, e.g. He Shou Wu, flavorings.

### EXAMPLE 6

A substrate-based process has been set up according to table 7.

As substrates were used in the order: Muira Puama wood tea leaves; Damiana tea leaves; Schisandra chinensis whole fruit; He Shou Wu e.s. 10-1;
A hydroalcoholic solution is used as a solvent consisting of:
- Water 850 gr
- Alcohol 96% 250 gr

**TABLE 7**

| ***Raw material*** | ***Botanical name*** | ***Family*** | ***Amount gr x 1 kg*** |
|---|---|---|---|
| Muira Puama wood tea leaves | Ptychopetalum Olacoides Benth. lignum | Olacaceae | 200 |
| Damiana tea leaves | Turnera Aphrodisiaca Willd. Folia | Turneraceae | 200 |
| Schisandra chinensis whole fruits | Schisandra chinensis baill. Fructus | Schisandraceae | 100 |
| He Shou Wu | Poligonum multiflorum thunb. Radix | Polygonaceae | 200 |

It then operates making a maceration of the muira puama in ultrasound for 24 hours 57 degrees. It is filtered, the residue is squeezed and then the solvent is used to extract damiana, 24 hours 57 degrees. The residue is filtered and squeezed and then the solvent is used to extract schisandra, 24 hours 57 degrees.

The residue is filtered and squeezed and then the solvent is used to extract he shou wu, 24 hours 57 degrees. This extract will then be finished with filtration and squeezing of the residue and can be used as an ingredient to compose the final product by mixing it with other ingredients (honey, e.g. guarana, water flavorings). With regard to this EXAMPLE 6, it is surprisingly observed that the "cascade" extraction evidently and significantly increases the extraction yield in bioactive components with antioxidant action.

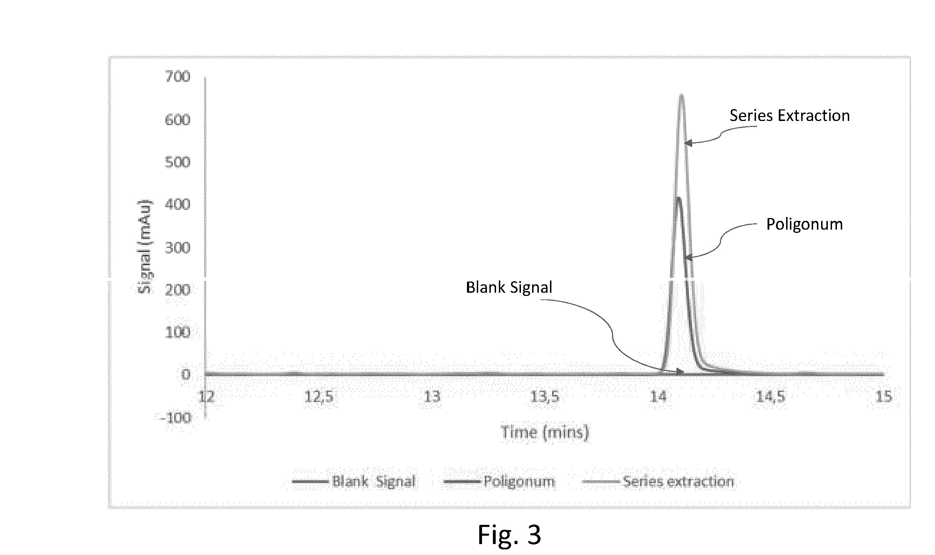

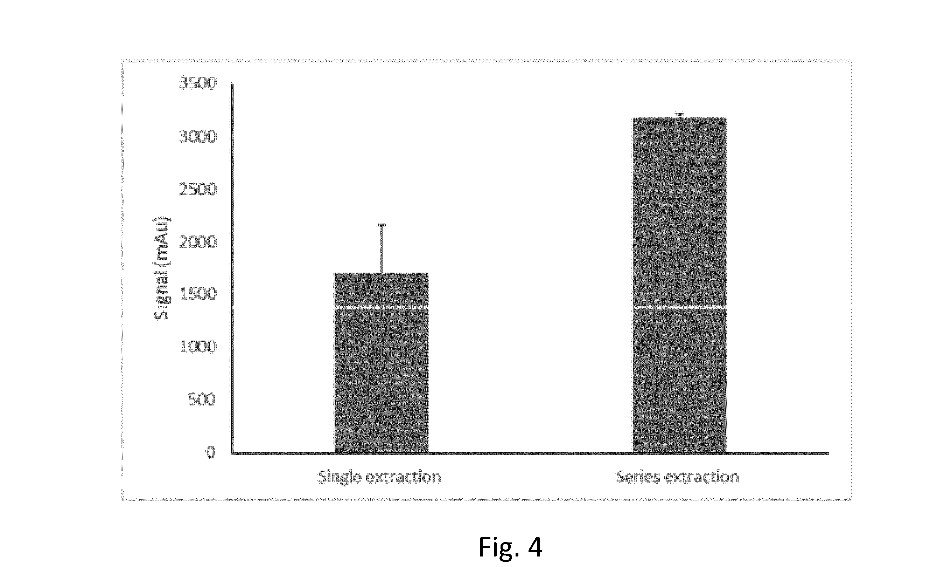

Figure 3 shows the result of a high-performance HPLC-type chromatography with a diode array detector for the extraction of Poligonum with a hydroalcoholic solution only, and with a solvent obtained from a "cascade" extraction of Ptychopetalum, Turnera and Schisandra . The results are also shown in figure 4.

### EXAMPLE 7

A substrate-based process was set up according to table 8.

The following were used as substrates: bearberry leaves, goldenrod at the top, malva silvestris flowers and leaves, hieracium pilosella plant.

A hydroalcoholic solution is used as a solvent consisting of:
- Water 700 gr
- Alcohol 96% 300 gr

**TABLE 8**

| ***Raw material*** | ***Botanical name*** | ***Family*** | ***Amount gr x 1 kg*** |
|---|---|---|---|
| BEARBERRY LEAVES | Arctostaphylos uva-ursi (L.) Sprengel | Ericacee | 500 |
| GOLDENROD AT THE TOP | Solidago virgaurea L. | Asteracee | 200 |
| MALVA SILVESTRIS FLOWERS AND LEAVES | Malva sylvestris L. | Malvaceae | 100 |
| HIERACIUM PILOSELLA PLANT | Hieracium pilosella L. | Asteraceae | 200 |

It then operates making a maceration of the pilosella in ultrasound for 24 hours 57 degrees. The residue is filtered, squeezed and then the solvent is used to extract gold rod, 24 hours 57 degrees. The residue is filtered and squeezed and then the solvent is used to extract mallow 24 hours 57 degrees. The residue is filtered and squeezed and then the solvent is used to extract bearberry into 2 parts (i.e. the plant is divided in half and 2 successive extractions are made), 24 hours 57 degrees. This extract will be finished with filtration and squeezing of the residue and can be used as an ingredient to compose the final product by mixing it with other ingredients.

### EXAMPLE 8

A method based on the substrates according to table 9 was prepared. The following were used as substrates: sempervivum, whole fresh plant, horsetail stem, cress.

A hydroalcoholic solution is used as a solvent consisting of:
- Birch sap 800 gr
- Alcohol 96% 200 gr

**TABLE 9**

| ***Raw material*** | ***Botanical name*** | ***Family*** | ***Amount gr x 1 kg*** |
|---|---|---|---|
| SEMPERVIVUM, WHOLE FRESH PLANT | Sempervivum tectorum L. | Crassulaceae | 500 |
| HORSETAIL STEM | Equisetum arvense L. | Equisetaceae | 300 |
| CRESS | Nasturtium officinale R. Brown | Brassicaceae | 200 |

It operates using the birch sap (collected by incision of the plant bark) as an extraction solvent on the sempervivum, after prior grinding with an immersion mixer, immersing the liquid obtained in the ultrasonic extractor for 24 hours 57 °.

It is filtered, the residue is squeezed and then the solvent is used to extract horsetail 24 hours 57 degrees.

The residue is filtered and squeezed and then the solvent is used to extract cress 24 hours 45 degrees.

This extract will be finished with filtration and squeezing of the residue and can be used as an ingredient to compose the final product by mixing it with other ingredients.

### EXAMPLE 9

A method based on the substrates according to table 10 was prepared.

As substrates, the following were used in order: Fennel whole fruit, Caraway whole fruit (Kummel), Angelica Root, Calamus natural rhizome, Fig Buds, Licorice Root. A hydroalcoholic solution is used as a solvent consisting of:
- Water 300 gr
- Alcohol 96% 700 gr

**TABLE 10**

| ***Raw material*** | ***Botanical name*** | ***Family*** | ***Amount gr x 1 kg*** |
|---|---|---|---|
| Fennel whole fruits | Foeniculum vulgare Miller | Apiaceae | 700 |
| Caraway whole fruit (Kummel) | Carum carvi L. | Apiaceae | 300 |
| Angelica Root | Angelica archangelica L. | Apiaceae | 200 |
| Natural rhizome aromatic calamus | Acorus calamus L. | Acoraceae | 100 |
| Fig Buds | Ficus carica L. | Moraceae | 100 |
| Licorice Root | Glycyrrhiza glabra L. | Leguminosae | 600 |

It then operates making a maceration of the liquorice in ultrasounds for 24 hours 57 degrees. It is filtered, the residue is squeezed and then the solvent is used to extract fennel, 24 hours 57 degrees. The residue is filtered and squeezed and then the solvent is used to extract caraway 24 hours 57 degrees. The residue is filtered and squeezed and then the solvent is used to extract angelica and calamus 24 hours 57 degrees. The residue is filtered and squeezed and then the solvent is used to extract fig buds 48 hours 40 degrees. This extract will be finished with filtration and squeezing of the residue and can be used as an ingredient to compose the final product by mixing it with other ingredients.

### EXAMPLE 10

A substrate-based process has been set up according to table 11. The following were used as substrates: angelica root, tabebuia bark (lapacho), papaya fruit.

A hydroalcoholic solution is used as a solvent consisting of:
- Aloe juice 650 gr
- Alcohol 96% 350 gr

**TABLE 11**

| ***Raw material*** | ***Botanical name*** | ***Family*** | ***Amount gr x 1 kg*** |
|---|---|---|---|
| ANGELICA ROOT | Angelica archangelica L. | Apiaceae | 300 |
| TABEBUIA BARK(LAPACHO) | Tabebuia avellanedae Lorenz | Bignoniaceae | 400 |
| PAPAIA FRUIT | Carica papaya L. | Caricaceae | 300 |

It then operates by making an angelic maceration starting from the aloe juice and alcohol in ultrasound for 48 hours at 45 degrees. The residue is filtered, squeezed and then the solvent is used to extract tabebuia, 48 hours 45 degrees. The residue is filtered and squeezed and then the solvent is used to extract papaya 48 hours 45 degrees, and it can be used as an ingredient to compose the final product by mixing it with other ingredients.

### EXAMPLE 11

In order to demonstrate the ameliorative properties of the final extract product obtained according to the process of this disclosure, tests were carried out.In particular, the antioxidant power of three traditional products was determined, and each of these three products was compared with a mixture of mother tinctures obtained by macerating the single medicinal plants that make up the components of the three products.

In particular, the following five samples were analyzed:
a) Sample according to the CASCADE mode (process of this disclosure);
b) Sample 01, 02, 03 wherein the components of the samples are obtained with a traditional system;
c) A reconstructed sample with single commercial extracts, i.e. with commercially available extracts.

In particular the samples 01, 02, 03 were composed as follows.

Sample 01 is obtained by separate extraction, under the same conditions as the sample according to the CASCADE embodiment, of the following plants: Crushed dried root Altea; Crushed dried top hyssop; Horehound crushed dried top; Pine Silvestris dried buds; Crushed dried Scutellaria Baicalensis; Dry extract of Pelargonium Sidoides.

Sulfur spring water, common water and vegetable glycerin were added to the extracts, according to the following table 12.

**TABLE 12**

| ***Raw material*** | ***Botanical name*** | ***Family*** | ***Amount gr x 1 kg*** |
|---|---|---|---|
| Sulfur spring water | | | 800 |
| Crushed dried root marshmallow | Althaea officinalis L. radix | Malvaceae | 12 |
| Crushed dried top hyssop | Hyssopus officinalis L. summitas | Lamiaceae | 5 |
| Horehound crushed dried top | Marrubium vulgare L. summitas | Lamiaceae | 5 |
| Pine Silvestris dried buds | Pinus sylvestris L. gemma | Pinaceae | 8 |
| Crushed dried Scutellaria Baicalensis | Scutellaria lateriflora L. herba | Lamiaceae | 10 |
| Dry extract of Pelargonium Sidoides | Pelagornium Sidoides Dc radix e.s. | Geraniacea e | 30 |
| Common water | | | 1200 |
| Vegetable Glycerin | | | 400 |

Samples 02, 03 are the same of sample 01 apart from the addition of 5% grapefruit seeds, inserted as a natural preservative (sample 02), 10% grapefruit seeds, inserted as a natural preservative (sample 03). Samples 01, 02, 03 (although they do not include the same components of the CASCADE sample) were chosen as significant traditional examples of samples considered to already have good antioxidant capacity.

An analysis of the antioxidant capacity for comparative purposes was carried out on the above 5 samples (CASCADE, samples 01, 02, 03 and reconstructed sample).

The antioxidant capacity of the 5 samples was measured by the DPPH assay (Sharma OP, Bhat TK, "DPPH antioxidant assay revisited". (2009), Food Chemistry, 113 (4): 1202, doi: 10.1016 / j.foodchem. 2008.08.008).

Table 12 below shows the results of the analysis of the antioxidant capacity using the DPPH assay. The results of the analyzes are expressed as the mean of two replicates, accompanied by the standard deviation.

**TABLE 12**

| Samples | Media, mg/mL | ±SD |
|---|---|---|
| Sample 01 | 8.17 | 0.56 |
| Sample 02 | 12.01 | 0.16 |
| Sample 03 | 6.82 | 0.43 |
| **CASCADE Sample** | **19.21** | **0.63** |
| Reconstructed sample | 3.27 | 1.28 |

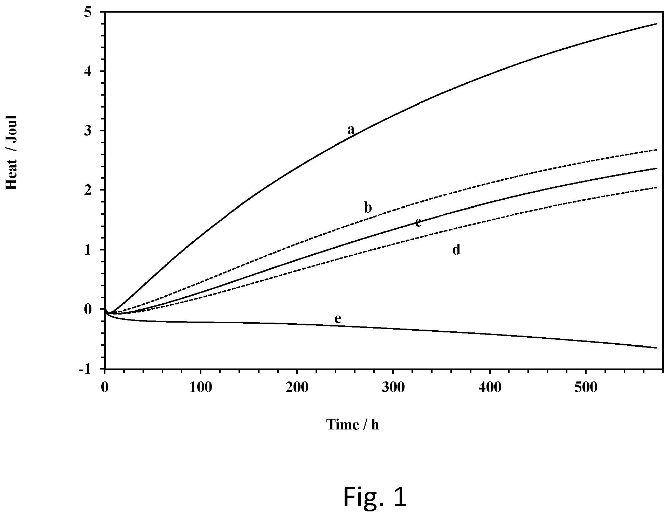

According to the results obtained, the CASCADE sample, made with the process according to the present disclosure, has the greatest antioxidant capacity compared to the extracts made with the traditional system (which as mentioned is considered good) and the extract reconstructed with the commercial extracts of the single plants. An analysis with isothermal calorimetry was also carried out. The results are shown in figure 1 here attached. In particular, figure 1 shows the value of the heat measured with the calorimetric procedure over time, wherein line a refers to sample of CASCADE mode, line b refers to sample 1, line c refers to sample 2, line d refers to sample 3 and the line e refers to the reconstructed extract.

The above five samples (CASCADE, samples 01, 02, 03 and reconstructed sample) were analyzed with the calorimetric procedure with the TAM III instrument (TA Instruments) at 40 °C according to the procedure developed in the UNIBZ laboratory (Haman N, Scampicchio M, et al. (2017) "A microcalorimetry study on the oxidation of linoleic acid and the control of rancidity" Talanta, 164: 407 doi: 10.1016 / j.talanta.2016.12.012).

The CASCADE sample (a) during the reaction with the oxygen in the ampoule inside the calorimeter at 40 °C generates the highest cumulative heat (5 J) compared to the other analyzed samples (b, c, d, e) . This result indicates the higher antioxidant power of the CASCADE sample (line a), i.e. of the sample obtained according to the process of the present disclosure.

In other words, the results of these tests highlighted the highest antioxidant capacity of the sample according to this disclosure in comparison with the extracts prepared with the traditional process and the extract reconstructed with commercial extracts.

## Claims

1. Extraction process for preparing a concentrated plant extract in hydroalcoholic solvent of at least three substrates of plant origin by means of extracting with the hydroalcoholic solvent a plurality of components from said at least three substrates of plant origin, wherein the three substrates are different from each other,
the process being a cascade extraction comprising:
at least a first extraction phase, a second extraction phase and a third extraction phase, wherein
the first extraction phase includes preparing a starting hydroalcoholic solvent and treating a first plant substrate with said starting hydroalcoholic solvent to transfer one or more first components of the first plant substrate into said hydroalcoholic solvent obtaining a first extraction product including said hydroalcoholic solvent and said one or more first components,
and collecting the first extraction product and using the first extraction product as a whole as primary extraction solvent,
the second extraction phase includes preparing said primary extraction solvent and treating a second plant substrate with said primary extraction solvent to transfer one or more second components of the second plant substrate into said primary extraction solvent, obtaining a second extraction product including said hydroalcoholic solvent and said one or more first components, and said one or more second components;
and collecting the second extraction product or second extract and using the second extraction product as a whole as secondary extraction solvent,
the third extraction phase includes preparing said secondary extraction solvent and treating a third plant substrate with said secondary extraction solvent to transfer one or more third components of the third plant substrate into said secondary extraction solvent, obtaining a third extraction product including said hydroalcoholic solvent and said one or more first components, said one or more second components and said one or more third components
and collecting said third extraction product or third extract; and wherein the first plant substrate is selected from the group including Brassica Oleracea Var Italica, Crataegus Oxyacantha auct., M, Glycyrrhiza Glabra L., Ptychopetalum Olacoides Benth, or a combination thereof, and
each of the second plant substrate and the third plant substrate is selected from the group including Birch pubescens, Brassica Oleracea Var Italica, Crataegus Oxyacantha auct., Eucommia Ulmoides Oliv., Filipendula. Ulmaria Max., Fraxinus Excelsior, Glycyrrhiza Glabra L., Harpagophytum Procubens D.C., Hippophae Rhamnoides L., Melissa Officinalis L., Moringa Oleifera Lamk, Olea Europaea L., Papaver Rhoeas L., Passiflora Incarnata L., Poligonum Multiflorum Benthalum Benthalum ThousandOth ., Rhodiola Rosea L., Salix Alba L., Schisandra Chinensis baill., Turnera Aphrodisiaca Willd., Urtica Dioica L., or a combination thereof;
such process allowing said first components, second components and third components to be concentrated by said cascade extraction in said starting solvent.

2. Process according to claim 1, including a subsequent extraction phase carried out on a fourth plant substrate using the third extraction product as a tertiary extraction solvent to obtain a fourth extraction product.

3. Process according to claim 2, wherein a further subsequent extraction phase is carried out in cascade mode onto a fifth plant substrate using the fourth extraction product of the previous extraction phase as an extraction solvent of said further subsequent extraction phase.

4. Process according to any one of the preceding claims, wherein said first plant substrate includes Crataegus oxyacantha auct., said second plant substrate includes Passiflora incarnata L., a third plant substrate includes Melissa Officinalis L., and the fourth plant substrate includes Rhodiola rosea L.

5. Process according to claim 3, wherein said first plant substrate includes Crataegus Oxyacantha auct., said second plant substrate includes Eucommia Ulmoides Oliv., the third plant substrate includes Urtica Dioica L., the fourth plant substrate includes Olea Europaea L., and the fifth plant substrate includes Papaver Rhoeas L.

6. Process according claim 3, wherein said first plant substrate includes Glycyrrhiza Glabra L., said second plant substrate includes Salix Alba L., the third plant substrate includes Filipendula Ulmaria Max., the fourth plant substrate includes Fraxinus Excelsior, and the fifth plant substrate includes Harpagophytum Procubens D.C.

7. Process according to claim 2, wherein said first plant substrate includes Ptychopetalum Olacoides Benth. lignum, said second plant substrate includes Turnera Aphrodisiaca Willd. Folia, the third plant substrate includes Schisandra chinensis baill. Fructus, and the fourth plant substrate includes Poligonum multiflorum thunb. Radix

8. Extraction product or extract obtained according to a process according to any one of the preceding claims, wherein the extraction product includes in a single final composition of said cascade extraction, at least said first components, second components and third components and said starting solvent.

9. A preparation including an extraction product according to claim 8 and further including any or a plurality of the following additional substances Licorice, honey, water, bioalcohol 96, or other ingredients.

## Patentansprüche

1. Extraktionsverfahren zum Herstellen eines konzentrierten Pflanzenextrakts in hydroalkoholischem Lösungsmittel aus mindestens drei Substraten pflanzlichen Ursprungs mittels Extrahieren mit dem hydroalkoholischen Lösungsmittel einer Vielzahl von Komponenten aus den mindestens drei Substraten pflanzlichen Ursprungs, wobei die drei Substrate voneinander unterschiedlich sind,
wobei das Verfahren eine Kaskadenextraktion ist, umfassend:
mindestens eine erste Extraktionsphase, eine zweite Extraktionsphase und eine dritte Extraktionsphase, wobei
die erste Extraktionsphase das Herstellen eines hydroalkoholischen Ausgangslösungsmittels und ein Behandeln eines ersten Pflanzensubstrats mit dem hydroalkoholischen Ausgangslösungsmittel einschließt, um eine oder mehrere erste Komponenten des ersten Pflanzensubstrats in das hydroalkoholische Lösungsmittel zu überführen, wobei ein erstes Extraktionsprodukt erhalten wird, das das hydroalkoholische Lösungsmittel und die eine oder die mehreren ersten Komponenten einschließt,
und Sammeln des ersten Extraktionsprodukts und Verwenden des ersten Extraktionsprodukts als Ganzes als primäres Extraktionslösungsmittel,
die zweite Extraktionsphase das Herstellen des primären Extraktionslösungsmittels und das Behandeln eines zweiten Pflanzensubstrats mit dem primären Extraktionslösungsmittel einschließt, um eine oder mehrere zweite Komponenten des zweiten Pflanzensubstrats in das primäre Extraktionslösungsmittel zu überführen, wobei ein zweites Extraktionsprodukt erhalten wird, das das hydroalkoholische Lösungsmittel und die eine oder die mehreren ersten Komponenten und die eine oder die mehreren zweiten Komponenten einschließt;
und Sammeln des zweiten Extraktionsprodukts oder zweiten Extrakts und Verwenden des zweiten Extraktionsprodukts als Ganzes als sekundäres Extraktionslösungsmittel,
die dritte Extraktionsphase das Herstellen des sekundären Extraktionslösungsmittels und das Behandeln eines dritten Pflanzensubstrats mit dem sekundären Extraktionslösungsmittel einschließt, um eine oder mehrere dritte Komponenten des dritten Pflanzensubstrats in das sekundäre Extraktionslösungsmittel zu überführen, wobei ein drittes Extraktionsprodukt erhalten wird, das das hydroalkoholische Lösungsmittel und die eine oder die mehreren ersten Komponenten, die eine oder die mehreren zweiten Komponenten und die eine oder die mehreren dritten Komponenten einschließt
und Sammeln des dritten Extraktionsprodukts oder dritten Extrakts; und wobei das erste Pflanzensubstrat ausgewählt ist aus der Gruppe einschließlich Brassica Oleracea Var Italica, Crataegus Oxyacantha auct., M, Glycyrrhiza Glabra L., Ptychopetalum Olacoides Benth, oder einer Kombination davon, und
jedes des zweiten Pflanzensubstrats und des dritten Pflanzensubstrats ist ausgewählt aus der Gruppe einschließlich Birch pubescens, Brassica Oleracea Var Italica, Crataegus Oxyacantha auct, Eucommia Ulmoides Oliv., Filipendula. Ulmaria Max., Fraxinus Excelsior, Glycyrrhiza Glabra L., Harpagophytum Procubens D.C., Hippophae Rhamnoides L., Melissa Officinalis L., Moringa Oleifera Lamk, Olea Europaea L., Papaver Rhoeas L., Passiflora Incarnata L., Poligonum Multiflorum Benthalum Benthalum ThousandOth ., Rhodiola Rosea L., Salix Alba L., Schisandra Chinensis baill., Turnera Aphrodisiaca Willd., Urtica Dioica L., oder einer Kombination davon;
wobei ein derartiges Verfahren ermöglicht, dass die ersten Komponenten, die zweiten Komponenten und die dritte Komponenten durch das Kaskadenextraktion in das Ausgangslösungsmittel konzentriert werden.

2. Verfahren nach Anspruch 1, das eine nachfolgende Extraktionsphase einschließt, die an einem vierten Pflanzensubstrat unter Verwendung des dritten Extraktionsprodukts als tertiäres Extraktionslösungsmittel durchgeführt wird, um ein viertes Extraktionsprodukt zu erhalten.

3. Verfahren nach Anspruch 2, wobei eine weitere nachfolgende Extraktionsphase in dem Kaskadenmodus an einem fünften Pflanzensubstrat durchgeführt wird, wobei das vierte Extraktionsprodukt der vorherigen Extraktionsphase als Extraktionslösungsmittel der weiteren nachfolgenden Extraktionsphase verwendet wird.

4. Verfahren nach einem der vorstehenden Ansprüche, wobei das erste Pflanzensubstrat Crataegus oxyacantha auct. einschließt, das zweite Pflanzensubstrat Passiflora incarnata L. einschließt, ein drittes Pflanzensubstrat Melissa Officinalis L. einschließt und das vierte Pflanzensubstrat Rhodiola rosea L. einschließt.

5. Verfahren nach Anspruch 3, wobei das erste Pflanzensubstrat Crataegus Oxyacantha auct. einschließt, das zweite Pflanzensubstrat Eucommia Ulmoides Oliv. einschließt, das dritte Pflanzensubstrat Urtica Dioica L. einschließt, das vierte Pflanzensubstrat Olea Europaea L. einschließt und das fünfte Pflanzensubstrat Papaver Rhoeas L. einschließt.

6. Verfahren nach Anspruch 3, wobei das erste Pflanzensubstrat Glycyrrhiza Glabra L. einschließt, das zweite Pflanzensubstrat Salix Alba L. einschließt, das dritte Pflanzensubstrat Filipendula Ulmaria Max. einschließt, das vierte Pflanzensubstrat Fraxinus Excelsior einschließt und das fünfte Pflanzensubstrat Harpagophytum Procubens D.C. einschließt.

7. Verfahren nach Anspruch 2, wobei das erste Pflanzensubstrat Ptychopetalum Olacoides Benth. lignum einschließt, das zweite Pflanzensubstrat Turners Aphrodisiacs Willd einschließt. wobei Folia, das dritte Pflanzensubstrat Schisandra chinensis baill einschließt. Fructus, und das vierte Pflanzensubstrat Poligonum multiflorum thunb. Radix einschließt.

8. Extraktionsprodukt oder Extrakt, das gemäß einem Verfahren nach einem der vorstehenden Ansprüche erhalten wird, wobei das Extraktionsprodukt in einer einzigen finalen Zusammensetzung der Kaskadenextraktion mindestens die ersten Komponenten, die zweiten Komponenten und die dritten Komponenten und das Ausgangslösungsmittel einschließt.

9. Präparat, das ein Extraktionsprodukt nach Anspruch 8 einschließt und ferner ein beliebiges oder eine Vielzahl der folgenden zusätzlichen Substanzen umfasst: Süßholz, Honig, Wasser, Bioalkohol 96 oder andere Inhaltsstoffe.

## Revendications

1. Procédé d'extraction permettant de préparer un extrait végétal concentré dans un solvant hydroalcoolique d'au moins trois substrats d'origine végétale au moyen d'une extraction avec le solvant hydroalcoolique d'une pluralité de composants provenant desdits au moins trois substrats d'origine végétale, dans lequel les trois substrats sont différents les uns des autres,
le procédé étant une extraction en cascade comprenant :
au moins une première phase d'extraction, une deuxième phase d'extraction et une troisième phase d'extraction, dans lequel
la première phase d'extraction comporte la préparation d'un solvant hydroalcoolique de départ et le traitement d'un premier substrat végétal avec ledit solvant hydroalcoolique de départ pour transférer un ou plusieurs premiers composants du premier substrat végétal vers ledit solvant hydroalcoolique en obtenant un premier produit d'extraction comportant ledit solvant hydroalcoolique et ledit ou lesdits premiers composants,
et la collecte du premier produit d'extraction et l'utilisation du premier produit d'extraction dans son ensemble en guise de solvant d'extraction primaire,
la deuxième phase d'extraction comporte la préparation dudit solvant d'extraction primaire et le traitement d'un deuxième substrat végétal avec ledit solvant d'extraction primaire pour transférer un ou plusieurs deuxièmes composants du deuxième substrat végétal vers ledit solvant d'extraction primaire, en obtenant un deuxième produit d'extraction comportant ledit solvant hydroalcoolique et ledit ou lesdits premiers composants, et ledit ou lesdits deuxièmes composants ;
et la collecte du deuxième produit d'extraction ou deuxième extrait et l'utilisation du deuxième produit d'extraction dans son ensemble en guise de solvant d'extraction secondaire,
la troisième phase d'extraction comporte la préparation dudit solvant d'extraction secondaire et le traitement d'un troisième substrat végétal avec ledit solvant d'extraction secondaire pour transférer un ou plusieurs troisièmes composants du troisième substrat végétal vers ledit solvant d'extraction secondaire, en obtenant un troisième produit d'extraction comportant ledit solvant hydroalcoolique et ledit ou lesdits premiers composants, ledit ou lesdits deuxièmes composants et ledit ou lesdits troisièmes composants
et la collecte dudit troisième produit d'extraction ou troisième extrait ; et dans lequel le premier substrat végétal est choisi dans le groupe comportant Brassica Oleracea Var Italica, Crataegus Oxyacantha auct., M, Glycyrrhiza Glabra L., Ptychopetalum Olacoides Benth, ou une combinaison de ceux-ci, et
chacun du deuxième substrat végétal et du troisième substrat végétal est choisi dans le groupe comportant Birch pubescens, Brassica Oleracea Var Italica, Crataegus Oxyacantha auct, Eucommia Ulmoides Oliv., Filipendula. Ulmaria Max., Fraxinus Excelsior, Glycyrrhiza Glabra L., Harpagophytum Procubens D.C., Hippophae Rhamnoides L., Melissa Officinalis L., Moringa Oleifera Lamk, Olea Europaea L., Papaver Rhoeas L., Passiflora Incarnata L., Poligonum Multiflorum Benthalum Benthalum ThousandOth., Rhodiola Rosea L., Salix Alba L., Schisandra Chinensis baill., Turnera Aphrodisiaca Willd., Urtica Dioica L., ou une combinaison de ceux-ci ;
un tel procédé permettant auxdits premiers composants, deuxièmes composants et troisièmes composants d'être concentrés par ladite extraction en cascade dans ledit solvant de départ.

2. Procédé selon la revendication 1, comportant une phase d'extraction ultérieure effectuée sur un quatrième substrat végétal en utilisant le troisième produit d'extraction en guise de solvant d'extraction tertiaire pour obtenir un quatrième produit d'extraction.

3. Procédé selon la revendication 2, dans lequel une phase d'extraction ultérieure supplémentaire est effectuée en mode cascade sur un cinquième substrat végétal en utilisant le quatrième produit d'extraction de la phase d'extraction précédente en guise de solvant d'extraction de ladite phase d'extraction ultérieure supplémentaire.

4. Procédé selon l'une quelconque des revendications précédentes, dans lequel ledit premier substrat végétal comporte Crataegus oxyacantha auct., ledit deuxième substrat végétal comporte Passiflora incarnata L., un troisième substrat végétal comporte Melissa Officinalis L., et le quatrième substrat végétal comporte Rhodiola rosea L.

5. Procédé selon la revendication 3, dans lequel ledit premier substrat végétal comporte Crataegus Oxyacantha auct., ledit deuxième substrat végétal comporte Eucommia Ulmoides Oliv., le troisième substrat végétal comporte Urtica Dioica L., le quatrième substrat végétal comporte Olea Europaea L., et le cinquième substrat végétal comporte Papaver Rhoeas L.

6. Procédé selon la revendication 3, dans lequel ledit premier substrat végétal comporte Glycyrrhiza Glabra L., ledit deuxième substrat végétal comporte Salix Alba L., le troisième substrat végétal comporte Filipendula Ulmaria Max., le quatrième substrat végétal comporte Fraxinus Excelsior, et le cinquième substrat végétal comporte Harpagophytum Procubens D.C.

7. Procédé selon la revendication 2, dans lequel ledit premier substrat végétal comporte Ptychopetalum Olacoides Benth. lignum, ledit deuxième substrat végétal comporte Turners Aphrodisiacs Willd. Folia, le troisième substrat végétal comporte Schisandra chinensis baill. Fructus, et le quatrième substrat végétal comporte Poligonum multiflorum thunb. Radix

8. Produit d'extraction ou extrait obtenu selon un procédé selon l'une quelconque des revendications précédentes, dans lequel le produit d'extraction comporte dans une unique composition finale de ladite extraction en cascade, au moins lesdits premiers composants, deuxièmes composants et troisièmes composants et ledit solvant de départ.

9. Préparation comportant un produit d'extraction selon la revendication 8 et comportant en outre l'une quelconque ou une pluralité des substances additionnelles suivantes : réglisse, miel, eau, bioalcool 96 ou d'autres ingrédients.
